# EUROPEAN PATENT SPECIFICATION

(11) **EP 2 641 623 B1**
(45) Date of publication and mention of the grant of the patent: **30.09.2020**
(21) Application number: 13156420.5
(22) Date of filing: 22.02.2013
(51) Int. Cl.: A61M 1/02, A01N 1/02, A61M 1/36, A61L 2/00, B04B 5/04

(54) **Apparatus and methods for providing cryopreserved ecp-treated mononuclear cells**
Vorrichtung und Verfahren zum Bereitstellen von kryokonservierten ECP-behandelten Mononuklearzellen
Appareil et procédés pour fournir des cellules mononucléaires traitées par ECP cryoconservées

(30) Priority: 20.03.2012 US 201261613239 P; 06.02.2013 US 201313760774
(43) Date of publication of application: 25.09.2013
(73) Proprietor: Fenwal, Inc., Lake Zurich, IL 60047 (US)
(72) Inventor: Min, Kyungyoon, Kildeer, IL 60047-8093 (US); Radwanski, Katherine, Des Plaines, IL 60016 (US); Heber, Cheryl, Hebron, IL 60034 (US)
(74) Representative: Prins, Hendrik Willem

(56) References cited:
- WO-A1-00/38762
- WO-A1-96/17514
- WO-A2-99/03976
- GB-A- 2 415 503
- US-A1- 2004 127 841
- US-A1- 2007 098 686

## Description

### Field of the Disclosure

The present disclosure relates to an apparatus and methods for providing cryopreserved mononuclear cells that have been treated by extracorporeal photopheresis ("ECP"). More particularly, the present disclosure relates to apparatus and methods for providing ECP-treated mononuclear cells that retain their apoptotic properties after cryopreservation and subsequent thawing.

### Background

Whole blood is made up of various cellular and non-cellular components such as red cells, white cells and platelets suspended in its liquid component, plasma. The administration of blood and/or blood components is common in the treatment of patients suffering from disease. Rather than infuse whole blood, it is more typical that individual components be administered to the patient as their needs require. For example, administration (infusion) of platelets is often prescribed for cancer patients whose ability to make platelets has been compromised by chemotherapy. Infusion of white blood cells (e.g. mononuclear cells), after the cells have undergone some additional processing or treatment, for example, to activate the mononuclear cells, may also be prescribed for therapeutic reasons including treatment of diseases that may involve the white blood cells. Thus, it is often desirable to separate and collect the desired blood component from whole blood and then treat the patient with the specific blood component. The remaining components may be returned to the donor or retained for other uses.

There are several diseases or disorders which are believed to primarily involve mononuclear cells, such as cutaneous T-cell lymphoma, organ allograft rejection after transplantation, graft versus host disease and autoimmune diseases such as rheumatoid arthritis, systemic sclerosis, among others, as described below.

Cutaneous T-cell lymphoma (CTCL) is a term that is used to describe a wide variety of disorders. Generally, CTCL is a type of cancer of the immune system where T-cells (a type of mononuclear cell) mutate or grow in an uncontrolled way, migrate to the skin and form itchy, scaly plaques or patches. More advanced stages of the disease also affect the lymph nodes. Therapeutic treatment options for CTCL have previously been limited. While chemotherapy has been utilized, this particular form of treatment also has many associated undesirable side effects such as lowered resistance to infection, bleeding, bruising, nausea, infertility and hair loss, just to name a few.

Organ allograft rejection may be characterized as the rejection of tissues that are foreign to a host, including transplanted cardiac tissue as well as lung, liver and renal transplants. Immunosuppression drug therapy following transplantation is common. However, there are potential drawbacks including recurring infection due to the compromised competence of the immune system caused by this type of therapy.

Similarly, graft versus host disease (GVHD) is a complication that can occur after a stem cell or bone marrow transplant in which the newly transplanted material attacks the transplant recipient's body. The differences between the donor's cells and recipient's tissues often cause T-cells from the donor to recognize the recipient's body tissues as foreign, thereby causing the newly transplanted cells to attack the recipient. GVHD may complicate stem cell or bone marrow transplantation, thereby potentially limiting these life-saving therapies. Therefore, after a transplant, the recipient is usually administered a drug that suppresses the immune system, which helps reduce the chances or severity of GVHD. See Dugdale, David C., et al. "Graft-Versus-Host Disease," MedlinePlus A.D.A.M Medical Encyclopedia, Updated June 2, 2010.

Autoimmune diseases, including rheumatoid arthritis (RA) and progressive systemic sclerosis (PSS), can be characterized by an overactive immune system which mistakes the body's own tissues as being a foreign substance. As a result, the body makes autoantibodies that attack normal cells and tissues. At the same time, regulatory T-cells, which normally function to regulate the immune system and suppress excessive reactions or autoimmunity, fail in this capacity. This may lead to, among other things, joint destruction in RA and inflammation of the connective tissue in PSS.

Where existing therapies for treating one or more diseases may result in certain unintended side effects, additional treatment may be desired or required. One known procedure which has been shown to be effective in the treatment of diseases and/or the side effects of existing therapies is extracorporeal photopheresis or "ECP". Extracorporeal photopheresis (also sometimes referred to as extracorporeal photochemotherapy) is a process that includes: (1) collection of mononuclear cells "MNC" from a patient, (2) photoactivation treatment of the collected mononuclear cells, and (3) reinfusion of the treated mononuclear cells back to the patient. More specifically, ECP involves the extracorporeal exposure of peripheral blood mononuclear cells combined with a photoactive compound, such as 8-methoxypsoralen or "8-MOP" which is then photoactivated by ultraviolet light, followed by the reinfusion of the treated mononuclear cells. It is believed that the combination of 8-MOP and UV radiation encourages and/or causes apoptosis or programmed cell death (also referred to herein as the "apoptosis trend") of ECP-treated cells.

Although the precise mechanism of action in ECP treatment (in the different disease states) is not fully known, according to early theories, it was believed that photoactivation causes 8-MOP to irreversibly covalently bind to the DNA strands contained in the T-cell nucleus. When the photochemically damaged T-cells are reinfused, cytotoxic effects are induced. For example, a cytotoxic T-cell or "CD8+ cell" releases cytotoxins when exposed to infected or damaged cells or otherwise attacks cells carrying certain foreign or abnormal molecules on their surfaces. The cytotoxins target the damaged cell's membrane and enter the target cell, which eventually leads to apoptosis or programmed cell death of the targeted cell. In other words, after the treated mononuclear cells are returned to the body, the immune system recognizes the dying abnormal cells and begins to produce healthy lymphocytes (T-cells) to fight against those cells.

In addition to the above, it has also been theorized that extracorporeal photopheresis also induces monocytes (a type of mononuclear cell) to differentiate into dendritic cells capable of phagocytosing and processing the apoptotic T-cell antigens. When these activated dendritic cells are re-infused into systemic circulation, they may cause a systemic cytotoxic CD8+ T-lymphocyte-mediated immune response to the processed apoptotic T-cell antigens like that described above. It will be appreciated that other possible mechanisms of action may be involved in achieving the benefits that have been observed from the ECP treatment of mononuclear cells and the subsequent benefits to patients undergoing ECP based therapies.

More recently, it has been postulated that ECP may result in an immune tolerant response in the patient. For example, in the case of graft versus-host disease, the infusion of apoptotic cells may stimulate regulatory T-cell generation, inhibit inflammatory cytokine production, cause the deletion of effective T-cells and result in other responses. See Peritt, "Potential Mechanisms of Photopheresis in Hematopoietic Stem Cell Transplantation," Biology of Blood and Marrow Transplantation 12:7-12(2006). While presently the theory of an immune tolerant response appears to be among the leading explanations, there exists other theories as to the mechanism of action of ECP relative to graft-versus-host disease, as well as other disease states.

In any event, it will be appreciated that ECP treatment directly promotes and encourages apoptosis of lymphocytes following exposure to UV light. Regardless of the precise mechanism of action, it is presently understood that apoptosis plays a role in the therapeutic properties achieved, and beneficial clinical effects of, ECP treatment.

Currently, known ECP treatment procedures (i.e. the apheresis collection of a mononuclear cell product from a patient, the addition of 8-MOP to the collected cell product, subsequent UV irradiation and the reinfusion of the treated mononuclear cell product) may be performed on two or more consecutive days on a weekly basis, the frequency depending on the state of the particular disease being treated and/or the patient's response to the treatment. Systems for performing ECP include, for example, the UVAR XTS Photopheresis System developed by Therakos, Inc., of Exton, PA. Further details of the Therakos system can be found, for example, in US Patent No. 5,984,887.

While the clinical benefits of ECP have been recognized, the use of ECP may be limited by logistical difficulties, including the need to repeatedly perform apheresis collections of mononuclear cells. Further, while methods for the cryopreservation of mononuclear cells prior to such ECP treatment has been described (see, for example, Merlin, E., et al. "Cryopreservation of mononuclear cells before extracorporeal photochemotherapy does not impair their antiproliferative capabilities." Cytotherapy 2011; 13:248-255), these methods would still require a physician or other clinician to perform the multiple steps of ECP treatment, including combining mononuclear cells with 8-MOP and irradiating the cells with UV light, each time the cryopreserved cells are thawed in order to obtain one or more therapeutic portions of ECP treated cells for re-infusion to a patient.

US2004/127841 A1 discloses a method for collecting a desired blood component and performing a photopheresis treatment. The method comprises mixing a photoactivation chemical with a separated blood component and after irradiation of the combination, at least the treated blood component is returned to the patient.

GB2415503 discloses recovery of stem cells from cord blood and may without pretreatment be stord by freezing followed by cryopreservation. The waste from which the stem cells are separated represent the pellet obtained after centrifugation of a cord blood sample, and comprising erythrocytes and multinuclear cells. The erythrocytes are lysed using a lysis buffer, which is also used for adjusting to osmomoality. Starting from cord blood samples, mononuclear cells may be obtained after centrifugation. The mononuclear cells are combinated with autologous plasma comprising 10% DMSO and frozen at a temperature of -80°C for overnight storage or in liquid N₂ for long time storage.

WO99/03976 discloses a method of treating leukocytes to arrest proliferation of the leukocytes and render them ineffective in eliciting graft-versus-host-disease (GVHD), but effective to enhance engraftment of allongeneic donor cells and promote destruction of diseased cells or pathogens. Thereto, as specified in claim 27, a cell population may be prepared by forming an *in vitro* reaction mixture comprising a population of leukocytes and a photochemical compound capable of forming a covalent bond with a nucleic acid, such as a psoralen. Subsequent the population is treated with UVA light, for instance at a light dose of 1 Joule/cm². The treatment is carried out such that a portion of the leukocyte population retains immunological activity.

The ability to cryopreserve substantially all, or a portion of, mononuclear cell products derived from an extracorporeal photopheresis treatment as described herein addresses these and other drawbacks. For example, the disclosed apparatus and methods allows for multiple portions of ECP treated mononuclear cell products to be collected from a single apheresis and photopheresis session, and allow the collected portions of ECP treated mononuclear cell products to be divided into smaller portions for more (or less) frequent administration while saving a patient from the burden of undergoing multiple or additional apheresis collections. It would also relieve the clinician from having to repeat the ECP treatment procedure each time cryopreserved untreated mononuclear cell products are thawed.

Therefore, it would be desirable to develop an apparatus and methods for storing an ECP-treated mononuclear cell product, such as by cryopreservation, which does not significantly affect the apoptosis trend of lymphocytes and/or other therapeutic properties or clinical benefits of ECP-based therapy after cryopreservation and the subsequent thawing. The steps of an ECP procedure would have to be performed less frequently, while allowing one or more therapeutic portions of cyropreserved ECP treated cell products to be obtained which may later be thawed and re-infused to a patient as part of a disease treatment protocol.

### Summary

In one aspect, the present disclosure is directed to a method of providing a cryopreserved treated mononuclear cell product as claimed in Claim 1. The method includes combining a mononuclear cell product with a photoactive compound that is activated by exposure to light of a selected wavelength. The mononuclear cell product combined with photoactive compound is then exposed to light for a selected period of time at the selected wavelength to obtain a treated mononuclear cell product. The method further includes combining therapeutic portions of the treated product with a cryopreservation medium and cryopreserving therapeutic portions of the treated mononuclear cell product.

In another aspect, the present disclosure is directed to a therapeutic portion of a cryopreserved treated mononuclear cell product present in a cryopreservation container as claimed in Claim 10. The treated product includes in one embodiment about 50 mL to about 300 mL of a mononuclear cell product that has been exposed to a selected dose of ultraviolet light and about 50 mL to about 300 mL of a cryopreservation solution. According to the invention, the mononuclear cell product is combined with a photoactive compound being 8-methoxypsoralen prior to exposure to ultraviolet light. According to the invention, the mononuclear cell product comprises about 100-300 nanograms/mL of 8-methoxypsoralen prior to exposure to ultraviolet light.

In another aspect, the present disclosure is directed to an apparatus for providing mononuclear cells that have been treated by extracorporeal photopheresis as claimed in Claim 12. The apparatus includes a disposable fluid circuit that has a processing chamber for separating whole blood into one or more components including mononuclear cells, at least one auxiliary storage container and a source of cryopreservation solution. The apparatus further includes a separation device adapted to receive the processing chamber for effecting separation of mononuclear cells from whole blood. The apparatus further comprises a source of a photoactive compound in openable flow communication with the disposable fluid circuit. The apparatus also includes at least one programmable controller that is programmed to separate mononuclear cells from whole blood and irradiate the cells with ultraviolet light to produce treated mononuclear cells and is further programmed to combine the treated cells with cryopreservation solution. The controller is also programmed to convey at least a portion of the treated cells that are combined with cryopreservation solution into the auxiliary storage container for cryopreservation therein. According to the invention, the disposable fluid circuit includes a source of 8-methoxypsoralen and/or a port adapted for introducing 8-methoxypsoralen into the fluid circuit and the controller may be further programmed to combine 8-methoxypsoralen with mononuclear cells prior to irradiating the cells with ultraviolet light.

### Brief Description of the Drawings

Fig. 1 is a diagram generally showing the mechanical components of an extracorporeal photopheresis treatment as described herein;
Fig. 2 is a flow chart setting forth the steps of the method of a photopheresis treatment including cryopreservation and thawing as described herein;
Fig. 3 is a partial perspective view of a separator useful in the methods and apparatus described herein;
Fig. 4 is a diagram of the fluid circuit useful in the collection, treatment and cryopreservation of mononuclear cells as described herein; and
Fig. 5 graphically illustrates the apoptosis trend of both fresh and cryopreserved/thawed ECP treated cells over a selected period of time.
Fig. 6 graphically illustrates the apoptosis trend of both fresh and cryopreserved/thawed ECP treated cells over a selected period of time.

### Description of the Illustrated Embodiments

The subject matter of the present disclosure relates generally to apparatus and methods for providing cryopreserved mononuclear cell products that have been treated by extracorporeal photopheresis (ECP). The cryopreserved treated mononuclear cell product may be thawed as needed for later reinfusion. As described herein, the therapeutic properties of the treated cell product is not significantly affected by cryopreservation or subsequent thawing.

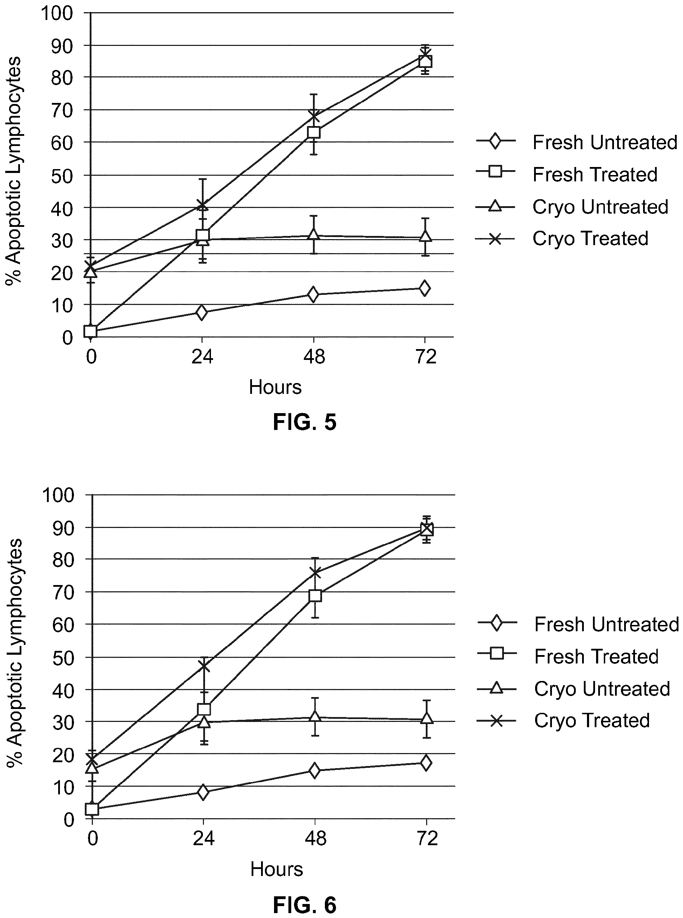

The term therapeutic properties as used herein includes, but is not limited to, the encouraged apoptosis or apoptosis trend of ECP treated cells, as described in further detail below. It will be appreciated that at least the apoptosis trend of cells treated in accordance with the apparatus and methods described herein is not significantly affected. "Not significantly affected" shall be understood to mean wherein the percentage of apoptotic cells of a given sample or therapeutic portion of mononuclear cells after ECP treatment, cryopreservation and subsequent thawing, is within ± 20% of the percentage of apoptotic cells of a sample or therapeutic portion of fresh (i.e. non-cryopreserved) ECP-treated cells over a given period of time. In other words, the percentage of apoptotic mononuclear cells following treatment in accordance with the disclosed apparatus and methods remains within 20% of ECP treated cells that are not subjected to cryopreservation and/or thawing over a selected period of time. If desired, it may be determined what percentage of cells have undergone apoptosis by performing certain tests on selected samples of treated and/or untreated cells as illustrated in Exemplary Figures 5 and 6 and as described in Examples A and B set forth below.

Turning now to one embodiment of the apparatus and methods described herein, mononuclear cells are provided. As used herein, "mononuclear cells" may also be referred to as "mononuclear cell product" or "MNC product". This may be accomplished by withdrawing whole blood from a patient, such as by an intravenous line or the like, and separating a mononuclear cell product from the whole blood by automated apheresis, centrifugation or other known automated or manual separation techniques. The mononuclear cell product may also be obtained from previously collected blood stored in a package, container or bag.

With regard to apheresis, the device in which the separation of blood occurs may include a centrifuge to provide a cell product comprising at least white blood cells. Non-limiting examples of apheresis devices that may be used to separate mononuclear cells from blood include the Alyx Separator and the Amicus® Separator made and sold by Fenwal, Inc. of Lake Zurich, Illinois. One example of an apparatus and method of collecting mononuclear cells is provided in U.S. patent no. 6,027,657. With regard to manual collection, whole blood may be collected in a bag or container and separated, such as by centrifugation, into its various component parts, including, for example, red blood cells, plasma (which may or may not contain platelets) and white blood cells. White blood cells may be retained in the bag while the remaining blood components can be manually expressed from the bag such as by squeezing or manipulation of the bag. It will be appreciated that white blood cells may also be obtained by bone marrow processing and/or from cord blood.

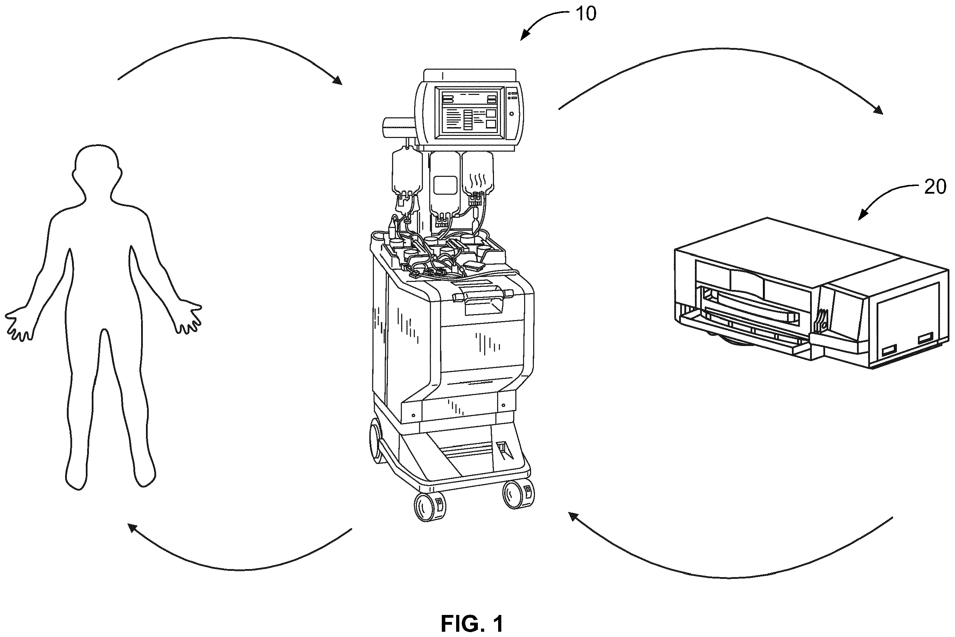

Fig. 1 shows, in general, the mechanical components that make up the exemplary apparatus and that are used in the methods described herein. In accordance with the present disclosure, the apparatus preferably includes a separation component 10 and a treatment (i.e., irradiation) component 20. A patient is connected to separation component 10 via a blood processing set, i.e., fluid circuit 200. With reference to Fig. 1, whole blood is withdrawn from the patient and introduced into the separation component 10 where the whole blood is separated to provide a target cell population. In a preferred embodiment in accordance with the present disclosure, the target cell population may be a mononuclear cell product. Other components separated from the whole blood, such as red blood cells and platelets may be returned to the patient or collected in pre-attached containers of the blood processing set 200 for storage or further processing.

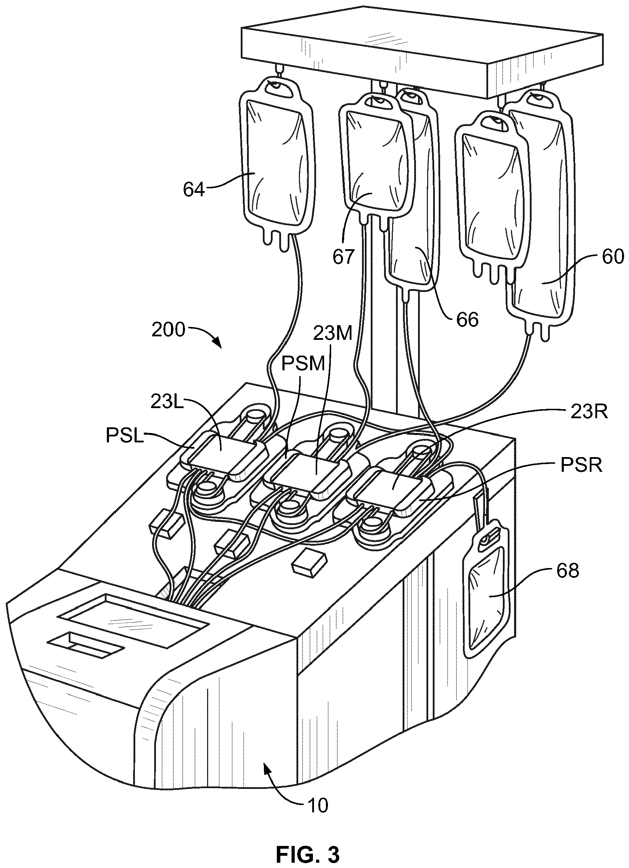

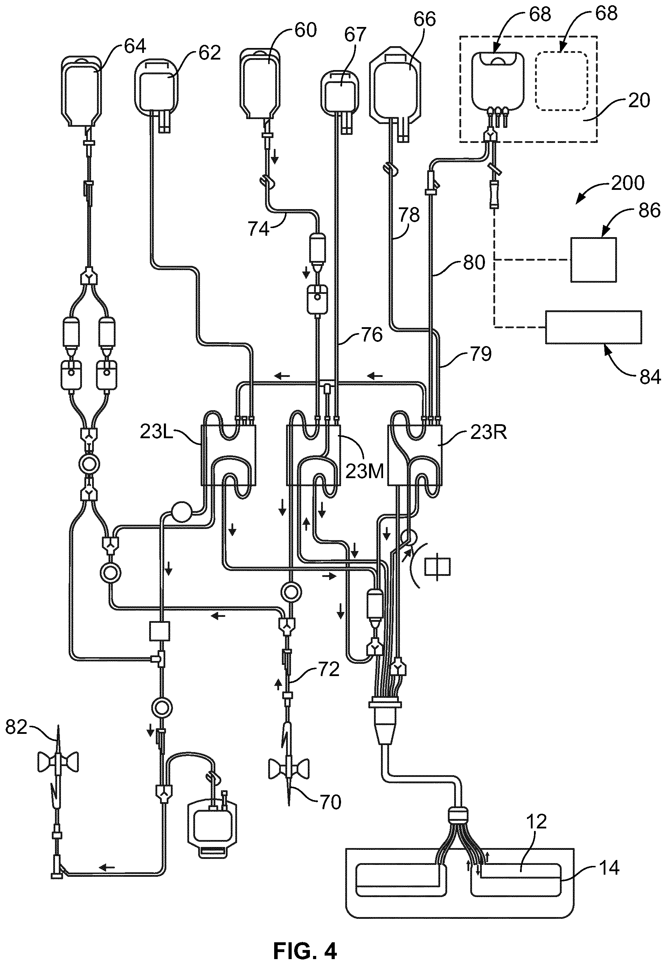

Briefly, Figs. 3-4 show a representative blood centrifuge 10 with fluid circuit 200 mounted thereon, the fluid circuit (Fig. 4) having a blood processing container 14 defining a separation chamber 12 suitable for harvesting a mononuclear cell product from whole blood. As shown in Fig. 3, a disposable processing set or fluid circuit 200 (which includes container 14) is mounted on the front panel of centrifuge 10. The processing set (fluid circuit 200) includes a plurality of processing cassettes 23L, 23M and 23R with tubing loops for association with peristaltic pumps PSL, PSM and PSR on device 10. Fluid circuit 200 also includes a network of tubing and pre-connected containers for establishing flow communication with the patient and for processing and collecting fluids and blood and blood components, as shown in greater detail in Fig. 4. As seen in Figs. 3 and 4, disposable processing set 200 may include a container 60 for supplying anticoagulant, a waste container 62 (see Fig. 4) for collecting waste from one or more steps in the process for treating and washing mononuclear cells, a container 64 for holding saline or other wash or resuspension medium, a container 66 for collecting plasma and one or more container(s) 68 for collecting, illuminating and/or cryopreserving the mononuclear cell product. Additional containers may be provided, for example, including a container 84 for holding a cryopreservation medium and/or a container 86 for holding a photoactive compound such as 8-MOP. Such containers may be integral with the fluid circuit 200 or provided separately.

With reference to Fig. 4, fluid circuit includes inlet line 72, an anticoagulant (AC) line 74 for delivering AC from container 60, an RBC line 76 for conveying red blood cells from separation chamber 12 of container 14 to container 67, a platelet-poor plasma (PPP) line 78 for conveying PPP to container 66 and line 80 for conveying mononuclear cells to and from separation chamber 12 and collection/illumination/cryopreservation container 68. As will be known to those of skill in the art, the blood processing set includes one or more venipuncture needle(s) for accessing the circulatory system of the patient. As shown in Fig. 4, fluid circuit 200 includes inlet needle 70 and return needle 82. In an alternative embodiment, a single needle can serve as both the inlet and outlet needle.

Fluid flow through fluid circuit 200 is preferably driven, controlled and adjusted by one or more microprocessor-based controllers in cooperation with the valves, pumps, weight scales and sensors of device 10 and fluid circuit 200, the details of which are described in the previously mentioned U.S. Patent No. 6,027,657. In one embodiment, the controller can be programmed to control various operations performed by the apparatus or device 10 disclosed herein. For example, the controller may be programmed to separate mononuclear cells from whole blood within the separation chamber 12 of the fluid circuit 200, combine the separated mononuclear cell product with a photoactive compound, irradiate mononuclear cell product with ultraviolet light in an illumination device 20 (which device may be associated with separation component 10 or separately provided therefrom) and/or combine the treated mononuclear cell product with a cryopreservation solution after irradiation. It will be appreciated that the controller may also be programmed to perform additional processing and treatment steps as necessary or desired.

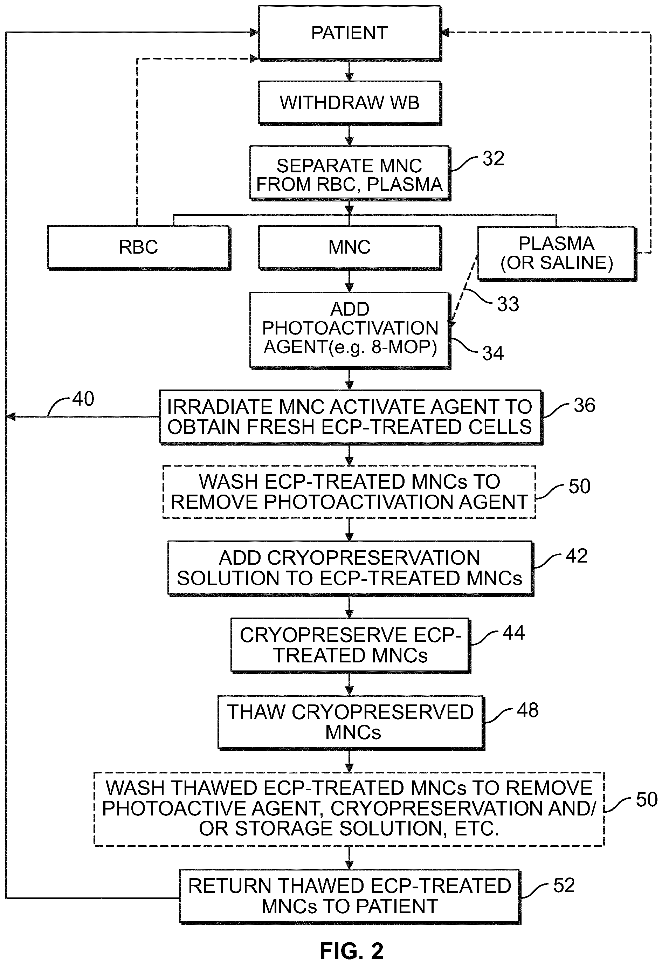

Collection of the mononuclear cell product may proceed in one or more cycles. The number of processing cycles conducted in a given therapeutic procedure will depend upon the total desired amount or portion of MNC to be collected. For example, in a representative procedure, five collection cycles may be performed sequentially. During each cycle about 1500-3000 ml of whole blood can be processed, obtaining a MNC product volume of about 3 ml per cycle and a total volume of 15 ml of MNC product. As shown in step 32 of Fig. 2, the final volume of collected mononuclear cell product is then provided for further treatment in accordance with the present disclosure. Of course, the collection of the MNC product is not limited to the method described above. MNCs may be collected in any manner known to those of skill in the art.

Effective treatment of the mononuclear cell product with light may require that the amount of collected mononuclear cells have a suitable hematocrit. Thereto the mononuclear cell product is diluted with a diluting solution such as plasma or saline, as shown in step 33. In the example described above, approximately 15 ml of MNC product may be diluted in an amount of plasma having a volume of about 1-300 ml and more preferably about 150 ml of plasma.

The diluted mononuclear cell product (in container 68) is then combined with the suitable photoactivation agent in step 34. As discussed above, for ECP treatment, the compound 8-methoxypsoralen (8-MOP) has been shown to be an effective photoactivation agent. It will be appreciated that mononuclear cell product may be combined with said photosensitive compound 8-MOP in any amount effective for satisfactorily treating cells by ECP. In one example, 8-MOP is combined with the collected and diluted mononuclear cell product to arrive at a mixture having a final 8-MOP concentration of 200 nanograms/mL and/or any suitable effective amount. According to the claimed method, the mononuclear cell product is combined with the photoactivation agent to arrive at a final 8-MOP concentration in a range of about 100 to 300 nanograms/mL.

The combination of 8-MOP and mononuclear cells can be accomplished by in vitro methods, such as by combining 8-MOP with a mononuclear cell product that has been collected by apheresis. In one example, the fluid circuit 200 (in which mononuclear cells are separated from whole blood) may include a source of 8-MOP in container 86. The 8-MOP or other photoactivation agent may be added directly to container 68 or added elsewhere into the fluid circuit 200, either manually by a syringe and/or under the direction of the microprocessor-based controller, which may be programmed to automatically deliver the desired amount of photoactive agent before, during, or after the MNC product collection, based on the volume of MNC product collected or to be collected. Alternatively, the desired volume of the agent may be pre-added to the container 68.

Alternatively, 8-MOP may be administered to a patient before whole blood is drawn and processed to separate mononuclear cells. In this method, 8-MOP may be administered in vivo, such as orally or intravenously. A portion of the blood from a patient to which 8-MOP has been administered may then be withdrawn and mononuclear cells separated therefrom.

Regardless of whether the mononuclear cell product and 8-MOP are combined in vivo or in vitro, the resulting combination of mononuclear cell product mixed with 8-MOP is preferably then exposed to or irradiated with light as shown in step 36 of Fig. 2. As noted above, the mononuclear cell product collected in accordance with the collection process described above may be collected in container 68 that is suitable for irradiation by light of a selected wavelength. By "suitable" it is meant that the walls of the container are sufficiently transparent to light of the selected wavelength to activate the photoactive agent. In treatments using UVA light, for example, container walls made of ethylene vinyl acetate (EVA) are suitable.

Accordingly, container 68 in which the mononuclear cell product is collected may serve both as the collection container and the irradiation container. Container 68 may be placed inside irradiation device 20 by the operator or more preferably, may be placed inside the irradiation chamber of irradiation device 20 at the beginning of the ECP procedure and prior to whole blood withdrawal (as shown by the broken lines representing device 20 in Fig. 4). In any event, container 68 may remain integrally connected to the remainder of fluid circuit 200 during the entire procedure, thereby maintaining the closed or functionally closed condition of fluid circuit 200. Alternatively, container 68 may be disconnected after mononuclear cell collection has been completed but before 8-MOP is added to the mononuclear cell product in container 68.

As noted above, the fluid circuit 200 is adapted for association with the treatment component (i.e., irradiation device) 20. It will also be appreciated, however, that the irradiation device does not have to be integral or even associated with the fluid circuit 200 and/or separation device 10. In fact, the irradiation device 20 may be in an entirely separate location from the separation device and/or circuit, such as a location in an entirely different room or building. In such a case, container 68 may be disconnected after collection has been completed for the later addition of 8-MOP to the mononuclear cell product in container 68 and/or irradiation of the container in one or more different locations.

One known apparatus suitable for the irradiation of mononuclear cells is available from sources such as Cerus Corporation, of Concord, California, such as, for example the irradiation device described in U.S. Patent No. 7,433,030. As shown and described in US Patent No. 7,433,030, irradiation device 20 preferably includes a tray or other holder for receiving one or more containers during treatment. Other irradiation devices may also be suitable for use with the method and apparatus described herein. However, it is also contemplated that suitable irradiation may also be accomplished by any source of ultraviolet light which provides UV light at a selected UVA dose, including natural sunlight.

Regardless of the type of irradiation device and/or the source of UV light, the mononuclear cell product combined with photoactivation agent (8-MOP) is irradiated for a selected UVA dose. In one non-limiting example, during treatment, the mononuclear cell product may be exposed to UV bulbs having a UVA wavelength in the UVA range of about 320 nm to 400 nm for a selected period of time, such as approximately 10-60 minutes, resulting in an average UVA exposure of approximately 0.5-5.0 J/cm² and preferably approximately 1-2 J/cm² or even more preferably approximately 1.5 J/cm2 per lymphocyte. As illustrated in Figure 2, following UV light exposure or irradiation of the mononuclear cell product combined with 8-MOP, the freshly treated cell product, or a portion thereof, may then be returned to the patient as illustrated by reference numeral 40.

It will be appreciated that the volume of mononuclear cell product obtained from a single apheresis collection and subsequently treated by extracorporeal photopheresis may provide more than one therapeutic portion of an ECP-treated mononuclear cell product. Accordingly, once a portion of freshly ECP treated mononuclear cell product is administered to a patient, there may be one or more portions of freshly treated mononuclear cell products remaining.

In accordance with the apparatus and methods described herein and as shown by step 44 in Figure 2, any portion of freshly treated cells that remain after a selected portion of freshly treated cells are administered to a patient, are cryopreserved. Alternatively, if none of the freshly ECP-treated cells are reinfused, then all of the freshly treated cells may preferably be cryopreserved in one or multiple separate containers for one or more future treatment sessions. Preferably, the ECP-treated cells are preserved by cooling to low sub-zero temperatures, such as by cryopreservation techniques at a temperature range of about -80°C to -200°C for example.

The cryopreservation of one or more portions of ECP-treated cells provides several advantages. For example, a typical ECP therapy schedule can be maintained (i.e. reinfusion of ECP-treated cells on one or more consecutive days) while reducing the number of repeated apheresis collections and UV irradiation procedures that must be performed. Another advantage is that ECP treated MNCs can be split or otherwise separated into smaller portions before cryopreservation and/or after thawing which can be administered more (or less) frequently, without subjecting the patient to an increased number of MNC collection procedures.

In accordance with the methods described herein, the ECP-treated mononuclear cell product is combined with a cryoprotectant solution or freezing media as illustrated by step 42 in Figure 2. In one embodiment, the treated mononuclear cell product and cryoprotectant is preferably combined in a ratio of 1:1. Any media suitable for the cryopreservation of mononuclear cells may be used. In one example, the cryopreservation media may contain human serum albumin, DMSO and/or starch. It will also be appreciated that the treated mononuclear cell product may optionally be combined with preservative and/or storage solutions including, but not limited to solutions containing bicarbonate, acetate, phosphate and/or citrate. Such solutions include RPMI developed by Moore et. al. at Roswell Park Memorial Institute and PAS V manufactured by Fenwal, Inc. The cryopreservation media and/or other preservative/storage solution may be added to the ECP-treated mononuclear cell product before (or contemporaneously with) conveying the treated cell product to a suitable cryopreservation container 68. Alternatively, the media may be added to the cells after they have been conveyed into one or more cryopreservation container(s) 68.

As shown in Fig. 4 fluid circuit 200 includes a source of cryopreservation media in, for example, container 84 in openable fluid communication with the circuit, such that cryopreservation media can be added directly to or combined with the treated mononuclear cell product in separation chamber 12 and/or in container(s) 68 or added elsewhere into the fluid circuit 200 either manually by a syringe and/or under the direction of the microprocessor-based controller which may be programmed to automatically deliver the desired amount of cryopreservation media. After being combined with cryopreservation media and/or a storage solution, the treated cell product is then cryopreserved in the one or more respective cryopreservation containers 68. In one example, container(s) 68 may be Cryocyte freezing bags sold by Baxter Healthcare of Deerfield, Illinois.

It will be appreciated that any known method suitable for the cryopreservation of a mononuclear cell product may be used. One such exemplary method of cryopreservation is described in Halle et al. "Uncontrolled-rate freezing and storage at -80°C, with only 3.5-percent DMSO in cryoprotective solution for 109 autologous peripheral blood progenitor cell transplantations", Transfusion, vol. 41, May 2001.

The treated MNC product may be cryopreserved for a selected period of time, ranging anywhere from several hours to several weeks or longer, up until a time when they are needed. In one example, the treated MNC product may be cryopreserved for one week, at which time a selected volume of treated cells, such as an amount sufficient to constitute a single therapeutic portion, is thawed as illustrated in Fig. 2, step 48, for administration to a patient. Of course, it will be appreciated that any desired amount of cryopreserved ECP-treated mononuclear cells may be thawed, as needed, for a particular therapeutic or treatment protocol.

Any suitable methods for thawing the treated cryopreserved mononuclear cell product may be used, including the procedure described in Halle et al., referenced above. In one example, the treated mononuclear cell product may be thawed rapidly in a 37°C water bath. It may be desirable to add a thawing media to the treated cell product during the thawing procedure. In one embodiment of the described method, the thawed ECP treated cells are suitable for immediate re-infusion to the patient. Alternatively, it is also contemplated that the thawed ECP-treated MNC product may be further processed before reinfusion.

In one embodiment, as shown in broken lines in step 50 Figure 2, such additional processing may include the optional washing of the treated MNC product prior to reinfusion. It may be desirable to wash the treated cells for several reasons, such as to remove photoactive agent and/or cryopreservation media from the treated mononuclear cells. More specifically, during photopheresis, MNCs are incubated with 8-MOP as the photoactive agent. Exposure of 8-MOP to (ultraviolet) light crosslinks the 8-MOP to the DNA and other proteins of MNCs, eventually resulting in cell apoptosis. However, not all of the 8-MOP crosslinks to the DNA of the MNCs. Some of the 8-MOP remains unbound and is infused to the patient with the treated cells. Excess (and unbound) 8-MOP is distributed throughout the body, making the patient especially sensitive to UV light exposure, particularly through the eyes. Therefore, as illustrated in Figure 2, it may be desirable to wash the ECP-treated MNC product to remove any excess and unbound photoactive agent from the treated MNC product prior to reinfusion, either before the treated cells are cryopreserved or after they are thawed. Other reasons for washing the treated MNC product prior to reinfusion to a patient may include to remove any storage or cryopreservation media that may have been added to the treated cell product prior to freezing.

Washing may be accomplished by centrifugation or other known washing techniques with washing media such as saline, RPMI and/or PAS V. Systems and methods that may be utilized to wash mononuclear cells in accordance with the present disclosure is described in US patent application Serial No. 13/733,607, filed on January 3, 2013, entitled "Apparatus and Methods for Providing Treated and Washed Mononuclear Cells". After the desired washing is complete, the washed mononuclear cell product may be returned or administered to a patient as needed as illustrated in step 52.

The aforementioned apparatus and methods are effective for providing one or more therapeutic portions of cryopreserved ECP-treated mononuclear cells useful in the treatment of one or more diseases and/or side effects of existing therapies. Stated differently, and as demonstrated in the examples below, MNCs prepared in accordance with the apparatus and methods described herein retain their apototic trend: when compared to freshly ECP-treated MNC products that have not been cryopreserved and/or thawed, the encouraged apoptosis or apoptosis trend of the treated mononuclear cell product after cryopreservation and subsequent thawing is not significantly affected. The following non-limiting examples are provided.

### EXAMPLE A

Purpose: To investigate cryopreservation of ECP treated and untreated mononuclear cells and to compare apoptosis levels in cultures made from frozen/thawed cells with that of cultures made from freshly collected cells.

Methods: Mononuclear cells were collected from healthy male donors using the Amicus Separator (Fenwal, Inc.) with the following settings: 2000 mL cycle volume, 12:1 WB to ACD-A ratio, MNC offset of 1.5 or 2.3 and RBC offset of 6.0 or 6.8 (n=8). The MNC product was diluted 1:1 with saline and incubated with 200 ng/mL 8-MOP (Sigma) for 15 minutes in the dark. An aliquot of cells was removed prior to irradiation to serve as an untreated control.

300 mL of diluted MNC product was irradiated for 10 minutes in an EVA bag (surface area 500 cm²) using the Therakos UVAR XTS device. Post irradiation, treated and untreated cells were purified using a Ficoll gradient. "Fresh" treated and untreated samples were resuspended for culture at 1 - 2 x 10⁶/mL in RPMI 1640 media supplemented with 2 mM glutamine and 10% human serum. "Cryo" treated and untreated samples were diluted 1:1 with freezing media (7% DMSO, 2% human serum in RPMI1640), aliquoted into cryovials and then cryopreserved with an uncontrolled rate freezer at -80°C.

After 1 week of cryopreservation, the cryovials were thawed rapidly in a 37°C water bath and washed with 500 U/mL DNase I and 1% human serum in PBS. The cells were washed twice with RPMI 1640 followed by culture at 1-2 x 10⁶/mL in RPMI 1640 media supplemented with 2 mM glutamine and 10% human serum. Fresh and cryopreserved/thawed cells were cultured at 37°C in a humidified chamber with 5% CO₂ for up to 72 hours. After 0, 24, 48 and 72 hours, samples were assayed for lymphocyte apoptosis. Apoptosis was measured as the % of CD45+/Annexin-V positive cells in the lymphocyte forward/side scatter gate.

The results of the aforementioned apoptosis measurements are shown in Figure 5, which graphically illustrates the % of apoptotic lymphocytes measured over 72 hours. It can be seen that the apoptosis trend of ECP treated cells that were cryopreserved and subsequently thawed was not significantly affected, as the percentage (%) of apoptotic lymphocytes is essentially the same as that for freshly ECP-treated cells over a 72 hour period.

### EXAMPLE B

The purpose and methods of Example B are essentially the same as Example A with the following exceptions: Example B was conducted on a larger scale than Example A such that cryo-bags were used in Example B in place of the cryo-vials used in Example A. Untreated and treated cells were frozen in Example B without Ficoll purification (n = 4). Treated and untreated cells were diluted 1:1 with freezing media (7 - 10% DMSO, 2% human serum in RPM11640) and frozen in EVA bags (70 - 80 mL total volume per 500 -750 mL cryobag). Post-thaw, the cells were washed with or without DNAse I and re-suspended for culture at 1 - 2 x 10⁶/mL in RPMI 1640 media supplemented with 2 mM glutamine and 10% human serum.

As with Example A and as shown in Figure 6, the apoptosis trend of ECP treated cells that were cryopreserved and subsequently thawed was not significantly affected. In other words, the percentage (%) of apoptotic lymphocytes of a mononuclear cell product after ECP treatment is within approximately 20% of the percentage of apoptotic cells of fresh (non-cryopreserved) ECP treated cells over a given period of time.

## Claims

1. A method of providing a cryopreserved treated mononuclear cell product comprising:
a) Diluting a mononuclear cell product with a diluting solution, wherein said mononuclear cell product has been obtained by separating whole blood into red blood cells, plasma and mononuclear cells in a separation device, with platelets being present with either the red blood cells or the plasma, and harvesting the mononuclear cells;
b) combining a mononuclear cell product with a photoactive compound that is activated by exposure to light of a selected wavelength, wherein the photoactive compound is 8-methoxypsoralen, which is present in a concentration of 100-300 ng/ml in the diluted mononuclear cell product
c) exposing the diluted mononuclear cell product combined with said photoactive compound to light for a selected period of time at the selected wavelength to obtain a treated mononuclear cell product comprising apoptotic cells,
d) splitting the treated mononuclear cell product into several therapeutic portions,
e) combining at least multiple or all of said portions of the treated mononuclear cell product with a cryopreservation medium, wherein the cryopreservation medium may be added to the treated mononuclear product before, contemporaneously with or after that a therapeutic portion thereof has been conveyed into a cryopreservation container;
f) cryopreserving the therapeutic portions of said treated mononuclear cell product that are conveyed into the cryopreservation containers, and
g) thawing a therapeutic portion of the cryopreserved mononuclear cell product in a cryopreservation container when needed, wherein the apoptosis trend of the treated cells is not significantly affected by the cryopreservation and subsequent thawing.

2. The method of claim 1, wherein the percentage of apoptotic cells after cryopreservation and subsequent thawing is within ±20% of the percentage of apoptotic cells of non-cryopreserved cells treated over a given period of time.

3. The method of claim 1 or 2 wherein said mononuclear cell product is derived from previously collected blood stored in a package, container or bag..

4. The method of claim 1 wherein the separation device is an automated apheresis separator.

5. The method according to any of the claims 1-4 further comprising the step of introducing said mononuclear cell product combined with said photoactive compound into a container, said container having walls at least a portion of which are transparent to light of a selected wavelength, prior to the step of exposing said product to light.

6. The method according to any of the claims 1-5 wherein said light has a wavelength in the ultraviolet spectrum in the range of 320 nm to 400 nm.

7. The method according to any of the claims 1-6, wherein the diluting solution is plasma or saline.

8. The method according to any of the claims 1-7, wherein the therapeutic portion is washed after thawing to remove the cryopreservation medium and any unbound photoactive compound.

9. The method as claimed in Claim 8, wherein the washing is accomplished by centrifugation with washing media.

10. A therapeutic portion of a cryopreserved treated mononuclear cell product present in a cryopreserved container and obtained by the steps a)-f) of the method as claimed in any of the claims 1-9.

11. The therapeutic portion of the cryopreserved treated mononuclear cell product as claimed in claim 10, comprising:
about 50 mL to about 300 mL of a mononuclear cell product combined with a photoactive compound being 8-methoxypsoralen, exposed to a selected dose of ultraviolet light, preferably about 0.5 J/cm² to about 5.0 J/cm², thus including apoptotic cells; and
about 50 mL to about 300 mL of a cryopreservation solution.

12. An apparatus for providing mononuclear cells that have been treated by extracorporeal photopheresis comprising:
(a) a disposable fluid circuit (200) comprising a processing chamber (12) for separating whole blood into one or more components including mononuclear cells,
(b) a source of photoactive compound (86) in openable flow communication with said fluid circuit (200),
(c) a separation device (10) adapted to receive said processing chamber (12) for effecting separation of said mononuclear cells from whole blood,
(d) a programmable controller programmed to:
i. separate mononuclear cells from said whole blood in said processing chamber (12),
ii. combine said mononuclear cells with the photoactive compound;
iii. irradiate said mononuclear cells combined with said photoactive compound with ultraviolet light to produce treated mononuclear cells,
**Characterized in that**:
- the disposable circuit (200) is further provided with multiple cryopreservation containers (68),
- the apparatus is provided with a source of cryopreservation medium (84) in openable flow communication with said fluid circuit (200),
- the programmable controller is further programmed to:
∘ split the treated mononuclear cells into several therapeutic portions,
∘ convey therapeutic portions of said treated mononuclear cells into said cryopreservation containers (68) for cryopreservation of said treated cells within said cryopreservation containers (68),
∘ add cryopreservation medium to said treated mononuclear cells, wherein cryopreservation medium is added to said therapeutic portions prior to, contemporaneously with or after conveying of the mononuclear cells.

## Patentansprüche

1. Verfahren zum Bereitstellen eines kryokonservierten behandelten Mononuklearzellenprodukts, das aufweist:
a) Verdünnen eines Mononuklearzellenprodukts mit einer Verdünnungslösung, wobei das Mononuklearzellenprodukt durch Trennen von Vollblut in rote Blutzellen, Plasma und Mononuklearzellen in einer Trennvorrichtung erhalten wird, wobei Plättchen entweder bei roten Blutzellen oder dem Plasma vorhanden sind, und Gewinnen der Mononuklearzellen;
b) Kombinieren eines Mononuklearzellenprodukts mit einer photoaktiven Zusammensetzung, die durch Aussetzen von Licht einer bestimmten Wellenlänge aktiviert wird, wobei die photoaktive Verbindung 8-Methoxypsoralen ist, das in einer Konzentration von 100-300 ng/ml im verdünnten Mononuklearzellenprodukt vorhanden ist;
c) Aussetzen des mit der photoaktiven Verbindung kombinierten verdünnten Mononuklearzellenprodukts von Licht für eine ausgewählte Zeitspanne in einer ausgewählten Wellenlänge, um ein behandeltes Mononuklearzellenprodukt erhalten, das apoptotische Zellen aufweist,
d) Aufteilen des behandelten Mononuklearprodukts in mehrere therapeutische Anteile,
e) Kombinieren von wenigstens mehreren oder allen der Anteile des behandelten Mononuklearzellenprodukts mit einem Kryokonservierungsmedium, wobei das Kryokonservierungsmedium zu dem behandelten Mononuklearprodukt vor, gleichzeitig mit oder nachdem ein therapeutischen Anteil davon in einen Kryokonservierungsbehälter übertragen wurde, hinzugefügt werden kann;
f) Kryokonservieren der therapeutischen Anteile des behandelten Mononuklearzellenprodukts, die in die Kryokonservierungsbehälter übertragen wurden, und
g) Auftauen eines therapeutischen Anteils des kryokonservierten Mononuklearzellenprodukts in einem Kryokonservierungsbehälter, wenn erforderlich, wobei die Apoptoseentwicklung der behandelten Zellen nicht wesentlich von der Kryokonservierung und dem nachfolgenden Auftauen betroffen ist.

2. Verfahren nach Anspruch 1, wobei der Prozentsatz der apoptotischen Zellen nach dem Kryokonservieren und dem nachfolgenden Auftauen bei ±20% des Prozentsatzes der apoptotischen Zellen von nicht-kryokonservierten Zellen, die über eine bestimmte Zeitspanne behandelt wurden, liegt.

3. Verfahren nach Anspruch 1 oder 2, wobei das mononukleare Zellprodukt aus zuvor gesammelten Blut gewonnen wird, das in einer Packung, einem Behälter oder einer Tasche aufbewahrt wird.

4. Verfahren nach Anspruch 1, wobei die Trennvorrichtung ein Apherese-Separator ist.

5. Verfahren nach einem der Ansprüche 1 bis 4, das weiterhin den Schritt des Einleitens des Mononuklearzellenprodukts in Kombination mit der photoaktiven Zusammensetzung in einen Behälter aufweist, und wobei der Behälter Wände aufweist, von denen wenigstens ein Teil davon durchlässig für Licht einer ausgewählten Wellenlänge ist, vor dem Schritt, dass das Produkt dem Licht ausgesetzt wird.

6. Verfahren nach einem der Ansprüche 1 bis 5, wobei das Licht eine Wellenlänge im ultravioletten Spektrum im Bereich von 320 nm bis 400 nm hat.

7. Verfahren nach einem der Ansprüche 1 bis 6, wobei die Verdünnungslösung Plasma oder salzhaltig ist.

8. Verfahren nach einem der Ansprüche 1 bis 7, wobei der therapeutische Anteil nach dem Auftauen gewaschen wird, um das Kryokonservierungsmedium und alle nicht gebundenen photoaktiven Verbindungen zu entfernen.

9. Verfahren nach Anspruch 8, wobei das Waschen durch Zentrifugieren mit Waschmedien erzielt wird.

10. Therapeutischer Anteil eines kryokonservierten behandelten Mononuklearzellenprodukts, das in einem kryokonservierten Behälter vorhanden ist und durch die Schritte a)-f) des Verfahrens nach einem der Ansprüche 1 bis 9 erhalten wird.

11. Therapeutischer Anteil des kryokonservierten behandelten Mononuklearzellenprodukts, nach Anspruch 10, der aufweist:
etwa 50 ml bis etwa 300 ml eines Mononuklearzellenprodukts kombiniert mit einer photoaktiven Verbindung, die 8-Methoxypsoralen ist, das einer ausgewählten Dosis von ultraviolettem Licht ausgesetzt wird, vorzugsweise etwa 0,5 J/cm² bis etwa 5,0 J/cm², somit apoptotische Zellen aufweisend; und
etwa 50 ml bis etwa 300 ml einer Kryokonservierungslösung.

12. Vorrichtung zum Bereitstellen von Mononuklearzellen, die mit einer extrakorporalen Photopherese behandelt wurden, die aufweist:
(a) einen entsorgbaren Fluidkreislauf (200), der eine Verarbeitungskammer (12) zum Trennen des Vollbluts in eine oder mehrere Komponenten einschließlich Mononuklearzellen aufweist,
(b) eine Quelle einer photoaktiven Verbindung (86) in einer öffenbaren Fließkommunikation mit dem Fluidkreislauf (200),
(c) eine Trennvorrichtung (10), die ausgelegt ist, die Verarbeitungskammer (12), um eine wirkungsvolle Trennung der Mononuklearzellen aus dem Vollblut zu erhalten, aufzunehmen,
(d) eine programmierbare Steuereinheit, die programmiert ist, um
i. Mononuklearzellen aus dem Vollblut in der Verarbeitungskammer (12) zu trennen,
ii. die Mononuklearzellen mit der photoaktiven Zusammensetzung zu kombinieren;
iii. die mit der photoaktiven Verbindung kombinierten Mononuklearzellen mit ultraviolettem Licht zu bestrahlen, um behandelte Mononuklearzellen herzustellen, **dadurch gekennzeichnet, dass**
- der entsorgbare Kreislauf (200) weiterhin mit mehreren Kryokonservierungsbehältern (68) vorgesehen ist,
- die Vorrichtung mit einer Quelle eines Kryokonservierungsmediums (84) in einer öffenbaren Fließkommunikation mit dem Fluidkreislauf (200) vorgesehen ist,
- die programmierbare Steuereinheit weiter dazu programmiert ist:
∘ die behandelten Mononuklearzellen in mehrere therapeutische Anteile aufzuteilen,
∘ die therapeutischen Anteile der behandelten Mononuklearzellen in die Kryokonservierungsbehälter (68) zum Kryokonservieren der behandelten Zellen in den Kryokonservierungsbehältern (68) zu übertragen,
∘ ein Kryokonservierungsmedium zu den behandelten Mononuklearzellen hinzuzufügen, wobei das Kryokonservierungsmedium zu den therapeutischen Anteilen vor, gleichzeitig oder nach dem Übertragen der Mononuklearzellen hinzugefügt wird.

## Revendications

1. Procédé de préparation d'un produit de cellules mononucléaires traitées, conservé par cryoconservation comprenant :
a) la dilution d'un produit de cellules mononucléaires avec une solution de dilution, dans lequel ledit produit de cellules mononucléaires a été obtenu en séparant du sang entier en globules rouges, plasma et cellules mononucléaires dans un dispositif de séparation, des plaquettes étant présentes soit avec les globules rouges soit le plasma, et en collectant les cellules mononucléaires ;
b) le mélange d'un produit de cellules mononucléaires avec un composé photo-actif qui est activé par exposition à un rayonnement d'une longueur d'onde sélectionnée, dans lequel le composé photo-actif est du 8-méthoxypsoralène, qui est présent en une concentration de 100 à 300 ng/ml dans le produit de cellules mononucléaires dilué,
c) l'exposition du produit de cellules mononucléaires dilué mélangé avec ledit composé photo-actif à un rayonnement de la longueur d'onde sélectionnée pendant une durée sélectionnée afin d'obtenir un produit de cellules mononucléaires traitées comprenant des cellules apoptotiques,
d) la division du produit de cellules mononucléaires traitées en plusieurs portions thérapeutiques,
e) le mélange d'au moins un multiple ou de la totalité desdites portions du produit de cellules mononucléaires traitées avec un milieu de cryoconservation, dans lequel le milieu de cryoconservation peut être ajouté au produit mononucléaire traité avant, simultanément ou après qu'une portion thérapeutique de celui-ci a été transférée dans un conteneur de cryoconservation ;
f) la cryoconservation des portions thérapeutiques dudit produit de cellules mononucléaires traitées qui sont transférées dans les conteneurs de cryoconservation, et
g) la décongélation d'une portion thérapeutique du produit de cellules mononucléaires conservé dans un conteneur de cryoconservation lorsque cela est nécessaire, dans lequel la tendance à l'apoptose des cellules traitées n'est pas sensiblement affectée par la cryoconservation et la décongélation ultérieure.

2. Procédé selon la revendication 1, dans lequel le pourcentage des cellules apoptotiques après cryoconservation et décongélation ultérieure est compris dans la plage de ±20% du pourcentage des cellules apoptotiques de cellules non conservées par cryoconservation traitées sur une durée donnée.

3. Procédé selon la revendication 1 ou 2, dans lequel ledit produit de cellules mononucléaires est dérivé de sang collecté préalablement, stocké dans un récipient, conteneur ou poche.

4. Procédé selon la revendication 1, dans lequel le dispositif de séparation est un séparateur par aphérèse automatique.

5. Procédé selon l'une quelconque des revendications 1 à 4, comprenant, en outre, l'étape d'introduction dudit produit de cellules mononucléaires mélangé audit composé photo-actif dans un conteneur, ledit conteneur comportant des parois dont au moins une partie sont transparentes au rayonnement d'une longueur d'onde sélectionnée, avant l'étape d'exposition dudit produit à un rayonnement.

6. Procédé selon l'une quelconque des revendications 1 à 5, dans lequel ledit rayonnement présente une longueur d'onde du spectre ultra-violet comprise dans la plage de 320 nm à 400 nm.

7. Procédé selon l'une quelconque des revendications 1 à 6, dans lequel la solution de dilution est du plasma ou une solution saline.

8. Procédé selon l'une quelconque des revendications 1 à 7, dans lequel la portion thérapeutique est lavée après décongélation afin d'éliminer le milieu de cryoconservation et tout composé photo-actif non lié.

9. Procédé selon la revendication 8, dans lequel le lavage est réalisé par centrifugation avec un milieu de lavage.

10. Portion thérapeutique d'un produit de cellules mononucléaires traitées, conservé par cryoconservation, présent dans un conteneur de cryoconservation et obtenue par les étapes a) à f) du procédé selon l'une quelconque des revendications 1 à 9.

11. Portion thérapeutique d'un produit de cellules mononucléaires traitées, conservé par cryoconservation selon la revendication 10, comprenant :
de 50 ml environ à 300 ml environ d'un produit de cellules mononucléaires mélangé à un composé photo-actif qui est du 8-méthoxypsoralène, exposé à une dose sélectionnée de rayonnement ultraviolet, de préférence, de 0,5 J/cm2 environ à 5,0 J/cm2, environ comportant ainsi des cellules apoptotiques ; et
de 50 ml environ à 300 ml environ d'une solution de cryoconservation.

12. Dispositif destiné à fournir des cellules mononucléaires qui ont été traitées par photophérèse extracorporelle comprenant :
(a) un circuit de fluide jetable (200) comprenant une chambre de traitement (12) destinée à séparer du sang total en un ou plusieurs composants comportant des cellules mononucléaires,
(b) une source de composé photo-actif (86) en communication d'écoulement pouvant être ouvert avec ledit circuit de fluide (200),
(c) un dispositif de séparation (10) adapté afin de recevoir ladite chambre de traitement (12) afin de réaliser la séparation desdites cellules mononucléaires par rapport au sang total,
(d) une unité de commande programmable, programmée de manière à :
i. séparer des cellules mononucléaires dudit sang total dans ladite chambre de traitement (12),
ii. mélanger lesdites cellules mononucléaires avec le composé photo-actif ;
iii. appliquer sur lesdites cellules mononucléaires mélangées audit composé photo-actif un rayonnement ultraviolet afin de produire des cellules mononucléaires traitées,
**caractérisé en ce que** :
le circuit jetable (200) comporte, en outre, de multiples conteneurs de cryoconservation (68),
le dispositif comporte une source de milieu de cryoconservation (84) en communication d'écoulement pouvant être ouvert avec ledit circuit de fluide (200),
l'unité de commande programmable est, en outre, programmée de manière à :
diviser les cellules mononucléaires traitées en plusieurs portions thérapeutiques,
transférer les portions thérapeutiques desdites cellules mononucléaires traitées dans lesdits conteneurs de cryoconservation (68) afin d'assurer la cryoconservation desdites cellules traitées à l'intérieur desdits conteneurs de cryoconservation (68),
ajouter un milieu de cryoconservation auxdites cellules mononucléaires traitées,
dans lequel le milieu de cryoconservation est ajouté auxdites portions thérapeutiques avant, simultanément ou après le transfert des cellules mononucléaires.
